# EUROPEAN PATENT APPLICATION

(11) **EP 2 537 476 A1**
(43) Date of publication of application: **26.12.2012**
(21) Application number: 11835900.9
(22) Date of filing: 20.06.2011
(51) Int. Cl.: A61B 17/28, A61B 17/06, A61B 17/3201

(54) **SURGICAL INSTRUMENT**

(30) Priority: 29.10.2010 JP 2010243022
(71) Applicant: Nakamura, Shoichi, Higashichikuma-gun Nagano 399-7502 (JP); ACP Japan Co. Ltd., Tokyo 113-0033 (JP)
(72) Inventor: NAKAMURA, Shoichi, Nagano 399-7502 (JP)
(74) Representative: Gassner, Wolfgang
(86) International application number: PCT/JP2011/064006
(87) International publication number: WO 2012/056759

(57) **Abstract**

The invention is to offer a surgical instrument, where the pressure-opposing force and bending characteristics of the spring parts can be easily adjusted and the manipulability is improved. This surgical instrument comprises functional parts 11 that perform nipping or cutting of the object, operating parts 13 that operate the functional parts 11 by a user adding force, and spring parts 14 composed of a pair of elastic strip-shaped parts that impel the operating part 13 in the direction of opening the functional parts 11, wherein multiple grooves 14A are formed in the strip-shaped members of the spring parts 14, thereby to cause changes in pressure-opposing force and in bending characteristics of the spring parts 14, and allowing adjustment of manipulability of the functional parts 11.

## Description

### Technical Field

The present invention relates to a surgical instrument, and in particular, to surgical gripper, needle holder, scissors, forceps, etc. for performing nipping or cutting of the object by using strip-shaped elastic members.

### Background Art

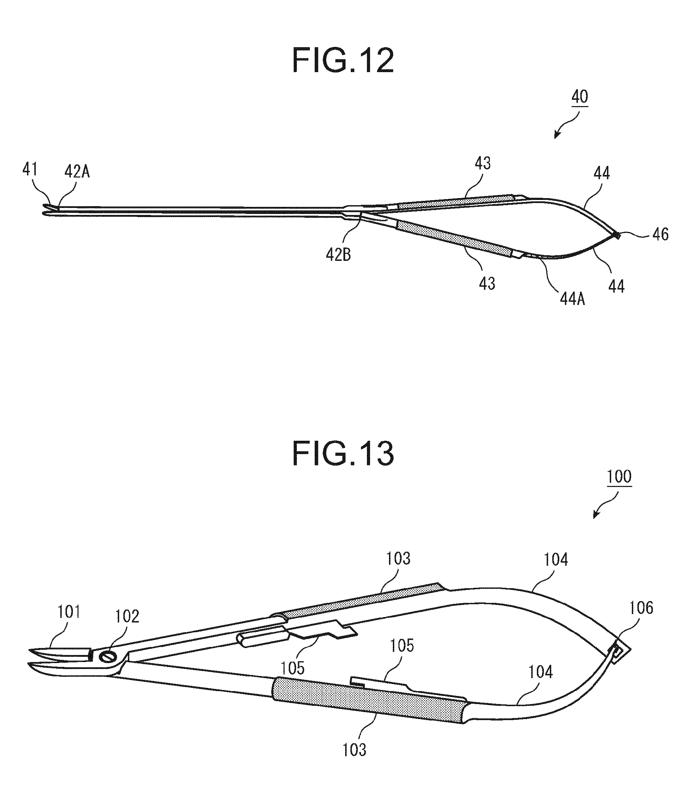

The surgical gripper, needle holder, scissors or forceps have spring parts composed of a pair of elastically strip-shaped members, and those have conventionally and broadly been used. FIG. 13 shows such a prior surgical instrument.

As shown in FIG. 13, the surgical instrument 100 (as an example herein, the needle holder is illustrated) is composed by joining, in X-shape, a one-side leg and another-side leg, and is provided with functional parts (nipping parts) 101 formed at the one-side ends of both legs, a fulcrum portion 102 pivoting both legs each other rotationally at their crossing portion, operating parts 103 enabling to open and close the functional parts 101 by a user holding these parts, spring parts 104 extending from the operating parts 103, engaging each other at their rear ends and impelling the operating parts 103 in their opening directions, and engaging parts 105 disposed at the insides of the operating parts 103 and enabling to engage with the operating parts 103 of both legs by matching a projecting strip-shaped part and a V-shaped part each other.

The one-side leg and the other-side leg thus composing the surgical instrument 100 are made of elastically metallic raw materials (titanium or stainless steels), and the functional parts 101, the operating parts 103 and the spring parts 104 are formed as one body.

Further, the surgical instrument 100 opens and closes the functional parts 101 by the user grasping the operating parts 103 and adding force thereto. The spring parts 104 are, as shown in FIG. 13, made of longer and thin strips, and are engaged each other in a manner that the one-side rear end and the other-side rear end are curved and engaged at a spring engaging portion 106, and are impelled in the directions of opening both legs.

By the way, if giving force to close the operating parts 103 more than at a certain interval, the functional parts 101 are closed and at the same time, the projecting strip-shaped portion of and the V-shaped portion of the engaging parts 105 are engaged each other and locked enabling to maintain the functional parts 101 closed. For canceling the engaging parts 105 from locking, force is further added in a direction of closing the operating parts 103 in order to release the strip-shaped part and the V-shaped part of the engaging parts 105.

### Summary of The Invention

### Problems to be Solved by The Invention

In the above mentioned existing surgical instrument 100, ordinarily, depending on the pressure-opposing force and the bending characteristic of the spring parts 104, a manipulability or a using perception (weight, hardness, etc. when using) when the user adds force are different. The pressure-opposing force and the bending characteristic are varied depending on the shape or raw material of the spring parts 104, and the manipulability or the using perception are influenced (for example, the titanium material feels hard).

In the case of the surgical instrument 100 having the above mentioned strip-shaped spring parts 104, an ideal is to give arbitrarily characteristics to a spring performance. That is, it is important to give pertinent the manipulability, irrespective of the shapes or the raw materials of the spring parts, and concurrently to adjust the suited using perception in response to respective embodiments such as gripper, needle holder, scissors, forceps, etc.

However, the present situation is that such delicate adjustment is difficult in the existing surgical instrument 100. Therefore, it has been difficult to consider a response to improvement of the manipulability, for example, such as an operation is heavy from closing of the functional parts 101 to locking of the engaging parts 105.

Therefore, in view of the above mentioned conventional problems, the present invention is to offer such a surgical instrument which enables to easily adjust the bending performance of the spring parts and to improve the manipulability.

### Means for Solving the Problem

For settling the above mentioned problems, the present invention is to offer such a surgical instrument which is provided with functional parts that perform nipping or cutting of the object, operating parts that operate the functional parts by the user applying force, and spring parts configured from a pair of elastically strip-shaped members and impelling the operating parts in the direction of opening the functional parts, and wherein multiple grooves are formed in the strip-shaped members of the spring parts, characterized by causing changes in the pressure-opposing force and in the bending characteristics of the spring parts, and allowing adjustment of manipulability of the functional parts.

Herein, the grooves are disposed in the strip-shaped members equally or irregularly in spaces. Further, it is desirable to form the grooves only in the insides of the strip-shapes, only in the outsides, or in both sides thereof. Further, preferably, the grooves are formed perpendicularly, otherwise obliquely with respect to the longer direction of the strip-shaped members.

By the way, the surgical instrument has desirably scissors composed of cutting edges in the functional parts. Or, the functional parts may be a needle holder composed of nipping parts.

The surgical instrument is composed by joining, in X-shape, a one-side leg and another-side leg, and at their crossing portion, a fulcrum portion is provided, pivoting both legs each other rotationally, and the functional parts are formed at the one sides of both legs while the operating parts and the spring parts are disposed at the other sides.

Otherwise, sufficiently, the surgical instrument is composed of the one-side leg and the other-side leg, where one sides of both legs are disposed with the functional parts, and the other sides are disposed with the operating parts and the spring parts, and the present surgical instrument may be provided with a first fulcrum point rotationally pivoting the one-side leg of the operating parts, a second fulcrum point rotationally pivoting the one-side leg of the functional parts, and a sliding shaft connecting the first and second fulcrum points and transmitting operation of the operating parts to the functional parts.

### Effects of The Invention

Depending on the surgical instrument of the invention, the pressure-opposing force and the bending characteristics of the spring parts are changed by a very simple processing only, so that it is possible to give a pertinent using perception, irrespective of the shapes or the raw materials of the surgical instrument, and concurrently adjust the above using perception to be a manipulability suitable in response to any embodiments such as gripper, needle holder, scissors, forceps, etc.

### Brief Description of Drawings

[FIG. 1] A perspective view showing the outline of the surgical instrument depending on one embodiment of the invention;
[FIG. 2] A perspective view showing the condition of locking the surgical instrument of FIG. 1 with the engaging parts; [FIG. 3] Typical views showing the details of the spring engaging parts in the surgical instrument of FIG. 1;
[FIG. 4] Typical views showing the outlines of the grooves formed in the surgical instrument of FIG. 1;
[FIG. 5] A diagram showing the examples of bending ratios in the points having the spring parts against pushing force (repulsive force) to the spring parts in the case of forming the grooves in the surgical instrument;
[FIG. 6] A typical view showing the grooves arranging example (No.1) of the surgical instrument depending on one embodiment of the invention;
[FIG. 7] Typical views showing the grooves arranging examples (No.2) of the surgical instrument depending on another embodiment of the invention;
[FIG. 8] A typical view showing the grooves arranging example (No.3) of the surgical instrument depending on a further embodiment of the invention;
[FIG. 9] Typical views showing the variation examples of the functional parts of the surgical instrument depending on the one embodiment of the invention;
[FIG. 10] A perspective view showing the outline of the surgical instrument (No.1) depending on another embodiment of the invention;
[FIG. 11] A perspective view showing the outline of the surgical instrument (No.2) depending on the other embodiment of the invention;
[FIG. 12] A perspective view showing the outline of the surgical instrument (No.3) depending on the further embodiment of the invention; and
[FIG. 13] A perspective view showing the outline of the prior surgical instrument (needle holder).

### Mode for Embodying The Invention

In the following description, explanations will be made to the embodiments of this invention, referring to the attached drawings.

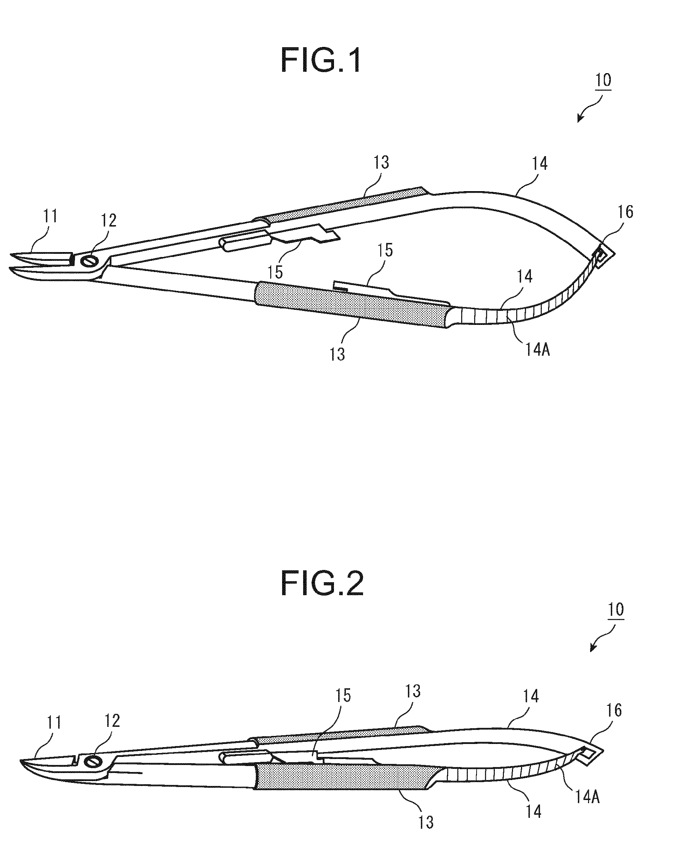

FIG. 1 is the perspective view showing the outline of the surgical instrument depending on the one embodiment of the invention. FIG. 2 is the perspective view showing the condition of locking this surgical instrument with the engaging part.

As shown in these Figures, the surgical instrument 10 (as an example herein, the needle holder is illustrated) is composed by joining, in X-shape, the one-side leg and the other-side leg, and is provided with nipping parts (functional parts) 11 formed at one end portions of both legs, a fulcrum portion 12 pivoting both legs each other rotationally at their crossing portion, operating parts 13 enabling to open and close the nipping parts 11 by a user holding these parts, spring parts 14 formed with multiple grooves 14A and impelling the operating parts 13 in the directions of opening the operating parts 13, and engaging parts 15 disposed at the insides of the operating parts 13 and enabling to engage with the operating parts 13 of both legs by matching a projecting strip-shaped portion with a v-shaped portion.

Both legs composing the needle holder 10 are made of a rarely rusting and elastic metallic material (titanium or stainless steels), where nipping parts 11, operating parts 13 and spring parts 14 are formed as one body. The rear ends of the nipping parts 11 through the operating parts 13 are the longer thin strip-shaped spring parts 14, and the rear portions of these spring parts 14 are engaged each other at a spring engaging portion 16.

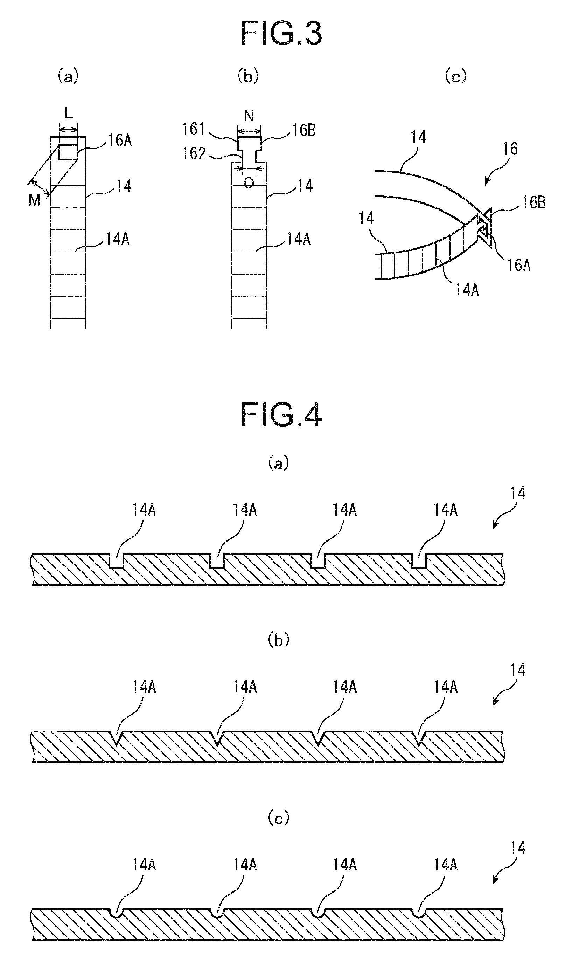

FIG. 3 shows in detail the spring engaging portion 16. FIG. 3(a) is the schematic view illustrating the rear end of the spring part of the one-side leg, FIG. 3(b) is the same illustrating the rear end of the spring part of the other-side leg, and FIG. 3(c) is the perspective view showing a condition of engaging both.

As shown in FIG. 3(a), the one-side leg has an open part 16A at the end in the spring part 14. As shown in FIG. 3(b), the other-side leg has an insertion part 16B at the end of the spring part 14. The insertion part 16B is composed of a head part 161 and a cervical part 162. The width N of the head part 161 is larger than the width L of the open part 16A, but smaller than a width across corner M. The width O of the cervical part 162 is smaller than the width L of the open part 16A. If getting an insertion part 16B into the open part 16A, an engagement can be made as FIG. 3(c) without dropping. By engaging the ends in such a way, the spring parts 14 of the longer curved thin strip impel both legs in the direction of opening both operating parts 13.

On the outsides of the spring parts 14, as shown in FIG.s 1 to 3, multiple grooves 14A are formed. FIG. 4 shows enlarged grooves 14A. FIG. 4(a) shows rectangular grooves, FIG. 4(b) shows v-shaped ones, and FIG. 4(c) shows semicircular grooves 14A. In this way, various shapes may be applied as the grooves 14A.

The strip-shaped spring part 14 made of a metal is easily curved at the portions forming the grooves 14A. By adjusting positions of forming the grooves 14A, spaces, groove number, etc., bending characteristics of the spring parts can be easily adjusted.

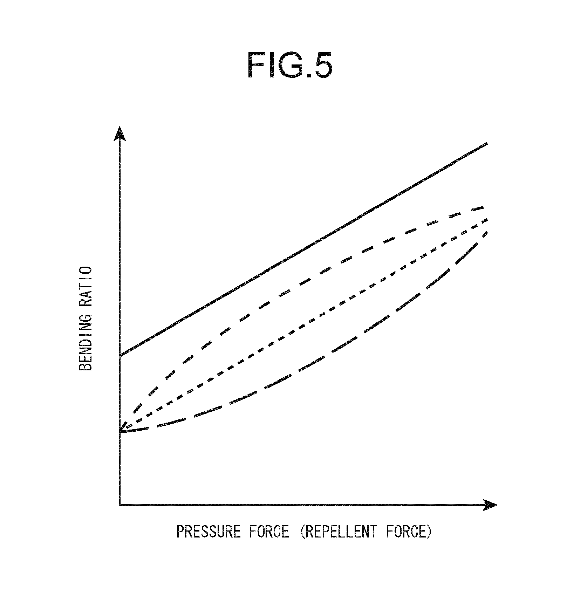

FIG. 5 is a graph showing the characteristics of the bending ratio in a certain one point of the spring parts 14 to the pressure force (repellent force of the spring part) against the spring parts 14. In FIG. 5, a solid line a shows the surgical instrument having the prior spring parts, and broken lines b, c and d show the surgical instrument having the spring parts of this invention.

As seen from FIG. 5, in the present invention, by forming the multiple grooves 14A at arbitrary positions of the spring parts 14, discretionary changes are available from the solid line a to the dotted lines b, c and d. By thus varying the bending characteristics of the spring parts 14, adjustment of the manipulability of the surgical instrument 10 can be made possible.

FIG.s 1 to 4 show the examples of disposing the grooves 14A at equal spaces only on the outsides of the spring parts 14, but for making adjustable the bending characteristics of the spring parts 14, the grooves 14A may be arranged at various spaces and at various positions.

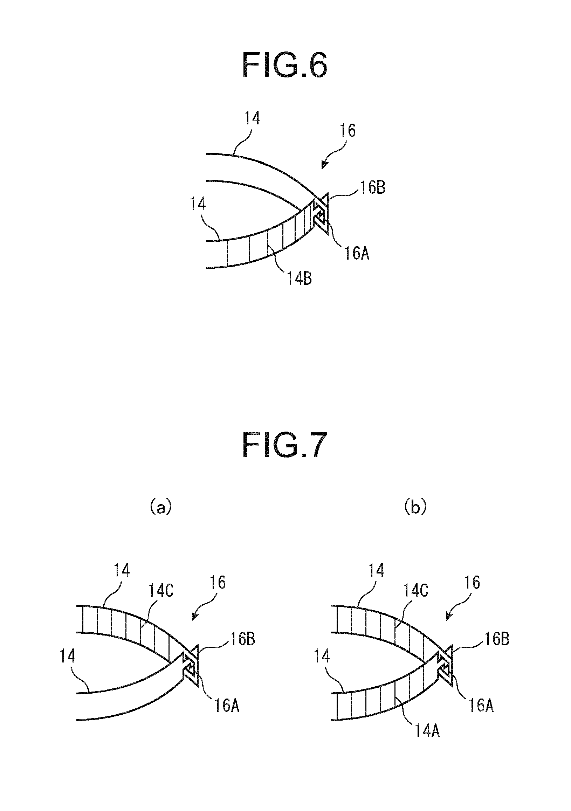

FIG. 6 shows an example of disposing the grooves 14B at non-equal spaces.

Further, FIG. 7 shows the grooves 14C placed only in the inside of the spring parts 14 [FIG. 7(a)], and the grooves 14A and 14C placed in both of the outside and the inside [FIG. 7 (b)].

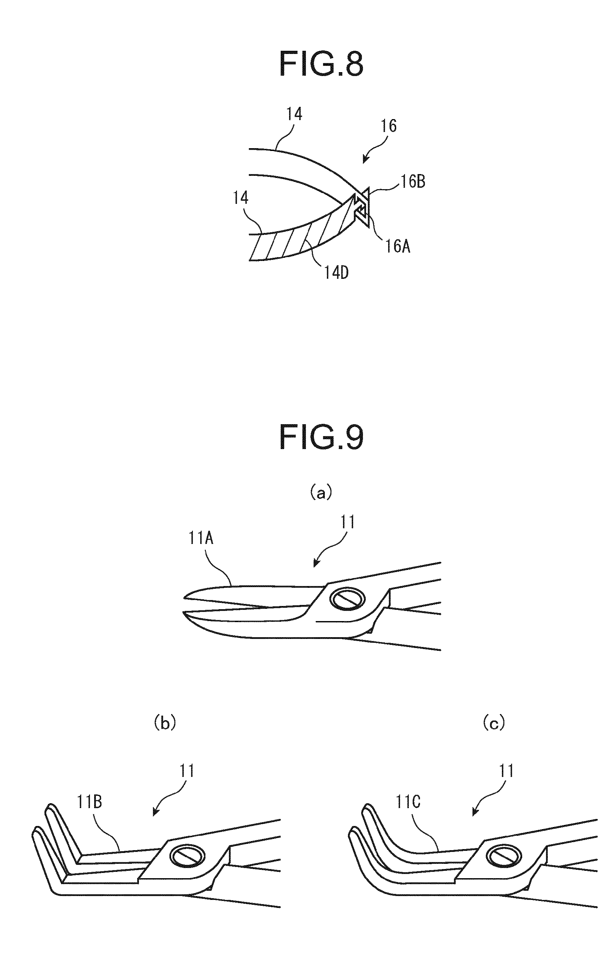

Furthermore, FIG. 8 shows an example of the grooves 14D formed obliquely with respect to the longer direction of the spring parts 14. In FIG.s 1 to 7, the grooves 14A, 14B and 14C are formed perpendicularly with respect to the longer direction of the spring parts 14 but not limiting to them, and various disposals may be provided.

In the above mentioned, the needle holder is exemplified as the surgical instrument 10, and if altering the embodiment of the functional parts 11, the invention can be applied to various surgical instruments such as scissors, especial forcipes, etc. Shapes of the functional parts 11 can be also deformed as straight or bending types.

FIG. 9 shows variation examples concerning the functional parts 11 of the surgical instrument 10. In FIG. 9(a), the functional parts 11 are the scissors, in FIG. 9(b), the functional parts 11 are the bent nipping 11B, and in FIG. 9(c), the functional parts 11 are the curved nipping 11C.

Further, the above embodiments have explained that the one-side leg and the other-side leg pivoted in X-shape are the surgical instrument 10 opening and closing by using the elastic strip-shaped member, and the spring parts 14 are placed at the rear ends of the functional parts 11 through the operating parts 13, that is, on the extension of the operating parts 13. However, the present invention is not limited thereto only, but can be applied to as far as being the surgical instrument which performs nipping or cutting by using the elastic strip-shaped member.

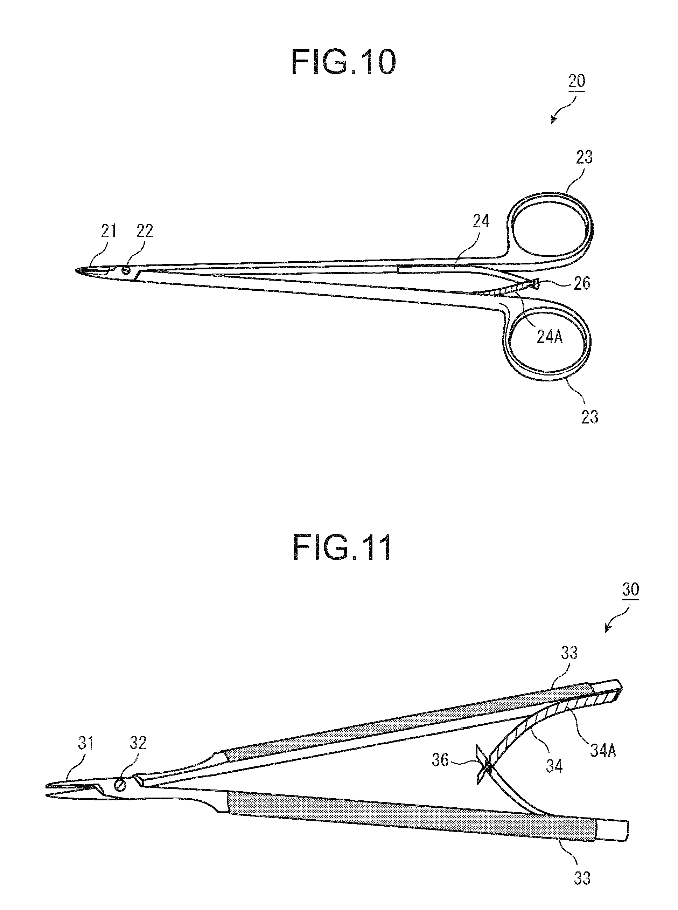

FIG. 10 shows the surgical instrument 20 having ring-shaped operating parts 23 (as an example herein, the scissors). This surgical instrument 20 is furnished with the functional parts (the cutting parts) 21, the fulcrum portion 22, the ring-shaped operating parts 23 and the multiple grooves 24A, and at the same time, furnished with the spring parts 24 positioned between the ring-shaped operating parts 23, and impelling the operating parts 23 in the opening direction. By the way, numeral 26 designates a spring engaging portion. Even the surgical instrument 20 of such an embodiment can adjust the manipulability by forming the grooves 24A in the spring parts 24.

FIG. 11 is the typical view showing the surgical instrument 30 having the spring parts 34 between the operating parts 33 (as an example herein, the needle holder). This surgical instrument 30 is furnished with the functional parts (the nipping parts) 31, the fulcrum portion 32, the operating parts 33 and the spring parts 34 formed with the multiple grooves 34A and at the same time positioned between the operating parts 33 and impelling the operating parts 33 in the opening direction. By the way, numeral 36 designates a spring engaging portion. Even the surgical instrument 30 of such an embodiment can adjust the manipulability by forming the grooves 34A in the spring parts 34.

FIG. 12 is the typical view showing the surgical instrument 40 (as an example herein, the needle holder) having a mechanism of opening and closing the functional parts 41 by means of a sliding shaft. This surgical instrument 40 has the functional parts (nipping parts) 41, the fulcrum portion 42A rotationally pivoting one of the operating parts 43, the fulcrum portion 42B rotationally pivoting one of the operating parts 43, the operating parts 43, the spring parts 44 formed with multiple grooves 44A and impelling the operating parts 43 in the opening direction, and the sliding shaft (not shown) connecting the functional parts 41 and the operating parts 43 via the fulcrum portions 42A, 42B. By the way, numeral 46 designates a spring engaging portion having a mechanism that if the user grasps the operating parts 43 and adds force to close the operating parts 43, the sliding shaft gears it and closes the functional parts 41, too. Even the surgical instrument 40 of such an embodiment can adjust the manipulability by forming the grooves 44A in the spring parts 44.

According to such a surgical instrument of this invention, if changing the pressure-opposing force and the bending characteristics of the spring parts by very simple process only, the pertinent using perception can be given, irrespective of the shapes or the raw materials of the surgical instrument, and even in any embodiments of nipping parts, needle holder, scissors, forceps, etc., they can be adjusted into proper manipulability.

The above references have explained the embodiments of the invention, but are not limited thereto, and so far as not deviating from the subject matter of the invention, various kinds of embodiments are, of course, available.

### Industrial Applicability

The invention relates to the surgical instrument, and in particular, to the surgical gripper, needle holder, scissors, forceps, etc. for performing gripping, cutting, etc. of the object by making use of the strip-shaped elastic parts.

### Description of Symbols

10: surgical instrument
11: functional parts (nipping parts)
11A: scissors
11B, 11C: nipping parts
12: fulcrum portion
13: operating parts
14: spring parts
14A, 14B, 14C: grooves
15: engaging parts
16: spring engaging portion
16A: open part
16B: insertion part
161: head part
162: cervical part
20: surgical instrument
21: functional parts (cutting parts)
22: fulcrum portion
23: ring-shaped operating parts
24: spring parts
24A: grooves
26: spring engaging portion
30: surgical instrument
31: functional parts (nipping parts)
32: fulcrum portion
33: operating parts
34: spring parts
34A: grooves
36: spring engaging portion
41: functional parts (nipping parts)
42A, 42B: fulcrum portions
43: operating parts
44: spring parts
44A: grooves
46: spring engaging portion

## Claims

1. A surgical instrument, which is provided with functional parts that perform nipping or cutting of the object, operating parts that operate the functional parts by the user applying force, and spring parts configured from a pair of elastically strip-shaped members and impelling the operating parts in the direction of opening the functional parts,
wherein multiple grooves are formed in the strip-shaped members of the spring parts, thereby to cause changes in pressure-opposing force and in bending characteristics of the spring parts, and allowing adjustment of manipulability of the functional parts.

2. The surgical instrument as set forth in claim 1, wherein the grooves are disposed in the strip-shaped members equally in spaces.

3. The surgical instrument as set forth in claim 1, wherein the grooves are disposed in the strip-shaped members irregularly in spaces.

4. The surgical instrument as set forth in claim 1, wherein the grooves are formed in the insides of the strip-shapes only.

5. The surgical instrument as set forth in claim 1, wherein the grooves are formed in the outsides of the strip-shapes only.

6. The surgical instrument as set forth in claim 1, wherein the grooves are formed in both of insides and outsides of the strip-shapes.

7. The surgical instrument as set forth in claim 1, wherein the grooves are formed perpendicularly with respect to the longer direction of the strip-shaped members.

8. The surgical instrument as set forth in claim 1, wherein the grooves are formed obliquely with respect to the longer direction of the strip-shaped members.

9. The surgical instrument as set forth in claim 1, wherein the surgical instrument has scissors composed of cutting edges in the functional parts.

10. The surgical instrument as set forth in claim 1, wherein the surgical instrument has a needle holder composed of nipping parts in the functional parts.

11. The surgical instrument as set forth in claim 1, wherein the surgical instrument is composed by joining, in X-shape, a one-side leg and another-side leg, and at their crossing portion, a fulcrum portion is provided, pivoting both legs each other rotationally, and the functional parts are formed at the one sides of both legs while the operating parts and the spring parts are disposed at the other sides.

12. The surgical instrument as set forth in claim 1, wherein the surgical instrument is composed of the one-side leg and the other-side leg, where one sides of both legs are disposed with the functional parts, and the other sides are disposed with the operating parts and the spring parts, and is provided with a first fulcrum point rotationally pivoting the one-side leg of the operating parts, a second fulcrum point rotationally pivoting the one-side leg of the functional parts, and a sliding shaft connecting the first and second fulcrum points and transmitting operation of the operating parts to the functional parts.
